# EUROPEAN PATENT APPLICATION

(11) **EP 4 635 946 A1**
(43) Date of publication of application: **22.10.2025**
(21) Application number: 24170724.9
(22) Date of filing: 17.04.2024
(51) Int. Cl.: C07D 295/135

(54) **IMPROVED PROCESS FOR THE PREPARATION OF CARIPRAZINE**

(71) Applicant: F.I.S. Fabbrica Italiana Sintetici S.p.A., 36040 Montecchio Maggiore (VI) (IT)
(72) Inventor: Rencurosi, Anna, 36040 Montecchio Maggiore (VI) (IT); De Poli, Matteo, 36040 Montecchio Maggiore (VI) (IT); Caputo, Francesco, 36040 Montecchio Maggiore (VI) (IT)

(57) **Abstract**

Object of the present invention is an improved process for the preparation of Cariprazine of formula (I): 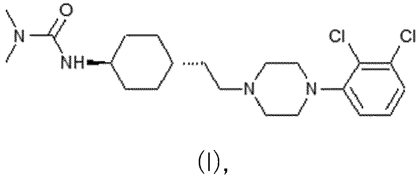 comprising the conversion of an imino-piperazine intermediate (II) to give an amino-piperazine intermediate by reaction with formic acid.

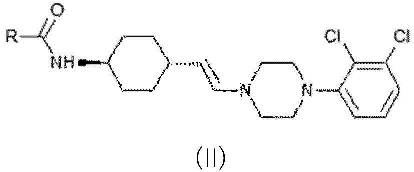

## Description

### Technical Field

The present invention refers to an improved process for the preparation of Cariprazine.

### Background Art

Cariprazine is a second generation antipsychotic used in the treatment of schizophrenia, bipolar mania, bipolar depression, and major depressive disorders, which was approved for medical use in the United States in September 2015.

Cariprazine is an N-alkylpiperazine that is N,N-dimethyl-N'-{trans-4-[2-(piperazin-1-yl)ethyl]cyclohexyl}urea substituted at position 4 on the piperazine ring by a 2,3-dichlorophenyl group.

Its chemical name is 3-[trans-4-[2-[4-(2,3-dichlorophenyl)piperazin-1-yl] ethyl]cyclohexyl]-1,1-dimethylurea and has the following chemical formula (I):

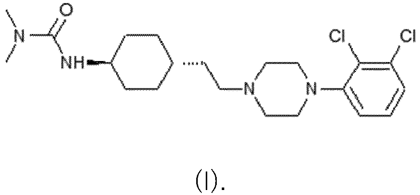

Cariprazine can be prepared according to several synthetic processes described in the literature.

The first synthesis of Cariprazine is described for example in WO 2005012266. The route of synthesis is reported in the following page. The synthesis starts with the reaction between 1-bromo-2,3-dichlorobenzene (1) and tertbutylpiperazine-1-carboxylate (2) using 2,2-bis-(diphenylphosphino)-1,1-binaphthyl (BINAP), tris-(dibenzylidene acetone)dipalladium(0) (Pd₂(dba)₃), and sodium tert-butoxide to afford tert-butyl-4-(2,3-dichlorophenyl)piperazine-1-carboxylate (3).

Intermediate (3) is then submitted to Boc deprotection with gaseous hydrogen chloride to give 1-(2,3-dichlorophenyl)piperazine (4). Next, reductive amination of tert-butyl-((1R,4R)-4-(2-oxoethyl)cyclohexyl) carbamate (5) with intermediate (4) using sodium triacetoxyborohydride in dichloromethane under alkaline conditions at room temperature for 20 h gives tert-butyl ((1R,4R)-4-(2-(4-(2,3-dichlorophenyl)-piperazin-1-yl)ethyl)cyclohexyl)carbamate (6). Intermediate 6, in turn, is reacted with gaseous hydrogen chloride to give (1R,4R)-4-(2-(4-(2,3-dichlorophenyl) piperazin-1-yl)ethyl)cyclohexanamine hydrochloride (7). Finally, the target compound 9 can be prepared following two sets of conditions. In the first set of conditions, compound (7) is treated with N,N-dimethylcarbamoyl chloride (8) to give Cariprazine (9). In the second set of conditions, compound (7) is reacted with triphosgene and dimethylamine hydrochloride to obtain Cariprazine (9).

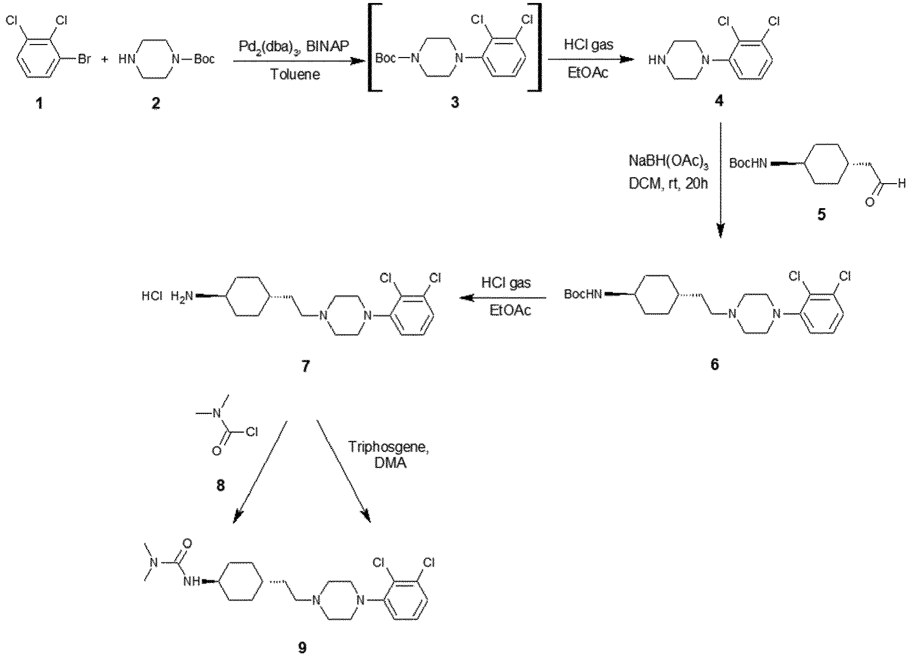

A new route of synthesis towards Cariprazine was later developed and disclosed in patent document CN 104496854, as depicted in the scheme attached in the following page. The synthesis starts with treatment of 4-aminocyclohexanone (10) with (8) using a phase-transfer catalyst to obtain 1,1-dimethyl-3-(4-oxocyclo hexyl)urea (11). This is followed by reaction on intermediate (11) affording ethyl 2-(4-(3,3-dimethylureido)-cyclohexylidene)acetate (13). Next, the compound ethyl trans-2-(4-(3,3-dimethylureido)cyclohexyl) acetate (14) is obtained by hydrogenation of compound (13). Subsequently, reduction of (14) using NaBH₄ affords 3-((1R,4R)-4-(2-hydroxyethyl)cyclohexyl)-1,1-dimethylurea (15).

Oxidation of intermediate (15) forms 1,1-dimethyl-3-(4-(2-oxoethyl) cyclohexyl)urea (16). Finally, reductive amination of intermediate (16) with piperazine (4) using sodium triacetoxyborohydride in dichloromethane under alkaline conditions yields Cariprazine (9).

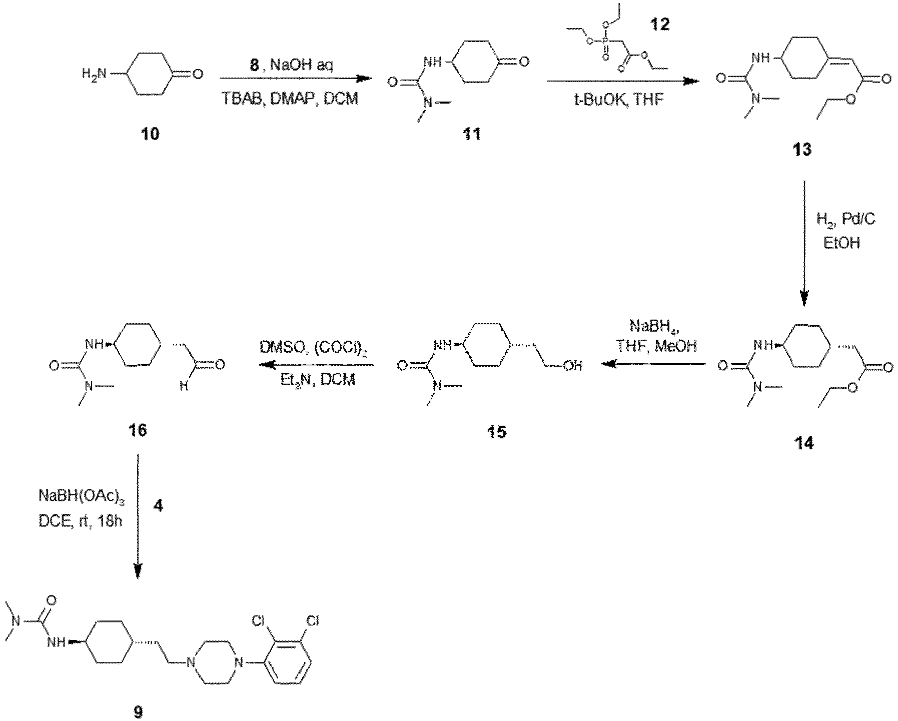

According to both the methods, a key intermediate aldehyde (intermediate (5) or intermediate (16) in the schemes reported above) is first reacted with piperazine intermediate (4) and the product obtained is then further reacted with sodium triacetoxyborohydride under alkaline conditions to give Cariprazine (9) or the N-protected Cariprazine precursor (6).

The reductive amination performed with sodium triacetoxyborohydride shows as a drawback a very long time required to complete the reaction. Specifically, 20 hours reaction time and 18 hours reaction time are reported in WO 2005012266 (Example 2) and in CN 104496854 respectively.

Furthermore, sodium triacetoxyborohydride is moisture sensitive, and thus difficult to handle, is quite an expensive reagent and generates byproduct wastes.

Thus, there is a need for a process for preparing Cariprazine wherein the reductive amination step is suitable for industrial manufacturing characterized by a shorter cycle time, thus improving the manufacturing process productivity, and a more sustainable process by reducing byproduct wastes.

### Summary of the invention

The problem addressed by the present invention is therefore that of providing a process for the preparation of Cariprazine suitable for industrial manufacturing therefore characterized by a shorter cycle time, improved productivity and, and the same time, improved sustainability (lower wastes).

This problem is solved by the annexed claims whose definitions are integral part of the present description.

Particularly, the present invention provides a process for the preparation of Cariprazine compound of formula (I): 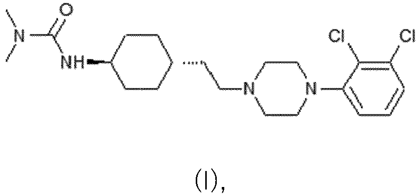 comprising the following steps:
a) reduction reaction of the compound of formula (II): 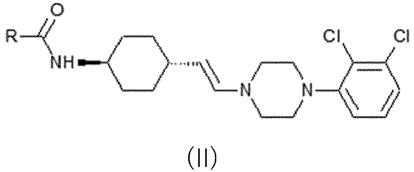 wherein R is O-R₁ or N(CH₃)₂ and wherein R₁ is C₁-C₆ linear or branched alkyl, phenyl or benzyl, with formic acid to give a compound of formula (III) : 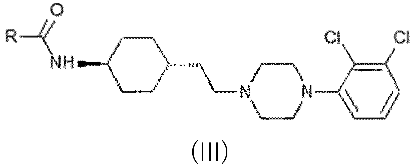 wherein R is O-R₁ or N(CH₃)₂ and wherein R₁ is C₁-C₆ linear or branched alkyl, phenyl or benzyl;
b) when R is not N(CH₃)₂, conversion of compound of formula (III) obtained in step a) to give Cariprazine compound of formula (I):

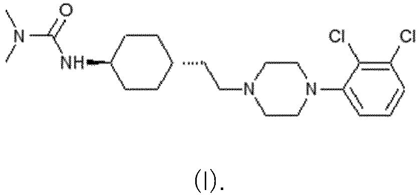

Another object is the use of formic acid for the preprataion of Cariprazine.

Further features and advantages of the processes according to the invention will result from the description hereafter reported of examples of realization of the invention, provided as an indication of the invention.

### Description of aspects

The present invention is related to a process for preparing Cariprazine compound of formula (I): 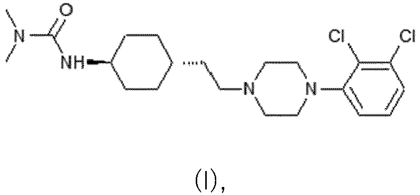 comprising the following steps:
a) reduction reaction of the compound of formula (II): 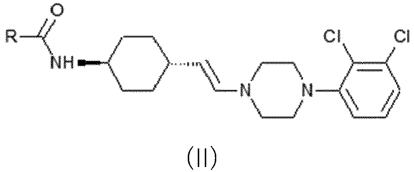 wherein R is O-R₁ or N(CH₃)₂ and wherein R₁ is C₁-C₆ linear or branched alkyl, phenyl or benzyl, with formic acid to give a compound of formula (III) : 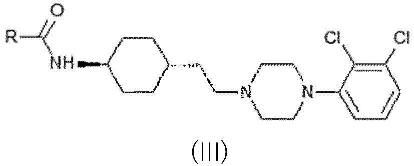 wherein R is O-R₁ or N(CH₃)₂ and wherein R₁ is C₁-C₆ linear or branched alkyl, phenyl or benzyl;
b) when R is not N(CH₃)₂, conversion of compound of formula (III) obtained in step a) to give Cariprazine compound of formula (I):

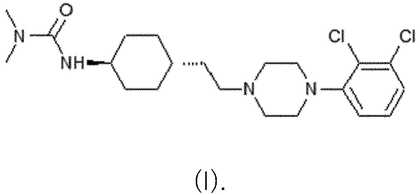

It was indeed surprinsingly found that using formic acid for the reduction of the double bond of compound of formula (II): 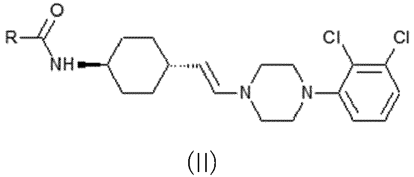 to give compound of formula (III): 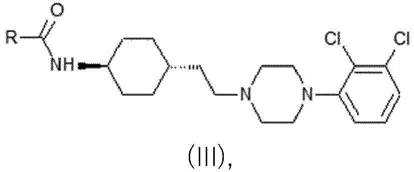 the reaction is typically complete after only 1 hour, whereas 18-20 hours of reaction time are reported in the literature (refer to Example 2 in WO 2005012266 and to CN 104496854) for the same reaction when sodium triacetoxyborohydride is employed as reactant, thus providing a more efficient or productive and sustainable process for preparing Cariprazine and solving the problem.

According to a preferred aspect of the process of the present invention, R is O-terbutyl.

As intended herein, the term C₁-C₆ linear or branched alkyl, means one alkyl group characterized by one to six carbon atoms, such as methyl, ethyl, 1-propyl, 2-propyl, 1-butil, 2-butyl, 2-methyl-1-propyl, 2-methyl-2-propyl, 1-pentyl, 1-hexyl etcetera.

According to a more preferred aspect of the process of the present invention, reduction reaction in step a) is carried out in a solvent.

According to an even more preferred aspect of the process of the present invention, reduction reaction in step a) is carried out in toluene, ethanol or 2-methyl-tetrahydrofuran.

According to a preferred aspect of the process of the present invention, in step a) the reaction is carried out at a temperature comprised in the range from 60° C to 80° C.

According to a more preferred aspect of the process of the present invention, in step a) the reaction is carried out at 75° C.

According to a preferred aspect of the process of the present invention, in step a) the reaction is carried out for a time comprised in the range from 30 minutes to 4 hours.

According to a more preferred aspect of the process of the present invention, in step a) the reaction is carried out for 1 hour.

According to a preferred aspect of the process of the present invention, in step a) the reaction is carried out with an amount of formic acid comprised in the range from 1 molar equivalent to 10 molar equivalents with respect to compound of formula (II).

According to a more preferred aspect of the process of the present invention, in step a) the reaction is carried out with an amount of formic acid comprised in the range from 3 molar equivalents to 5 molar equivalents with respect to compound of formula (II).

According to an even more preferred aspect of the process of the present invention, in step a) the reaction is carried out with 4.2 molar equivalents of formic acid with respect to compound of formula (II).

Molar equivalent or equivalent (eq) means that the molar amount of a substance reacts with a molar amount of another substance in a given chemical reaction.

According to a preferred aspect of the process of the present invention, in step a) the reaction is carried out in the presence of an inorganic acid, or an inorganic acid is added into the reaction mixture.

According to a preferred aspect of the process of the present invention, in step a) the reaction is carried out in the presence of hydrochloric acid, or hydrochloric acid is added into the mixture.

According to a preferred aspect of the process of the present invention, in step a) formic acid is added as a solution in an organic solvent.

According to a more preferred aspect of the process of the present invention, in step a) formic acid is added as a solution in 2-methyl-tetrahydrofuran.

According to an even more preferred aspect of the process of the present invention, in step a) formic acid is added neat into the mixture.

According to a preferred aspect of the process of the present invention, between step a) and step b) is comprised an additional step a1) comprising a re-crystallization or re-slurry of compound of formula (III) in an organic solvent.

By re-crystallization it is intended that a solid compound is dissolved in a solvent and then precipitated for example by cooling the solution and/or adding another solvent. The solid obtained is then usually isolated by filtration or centrifugation.

By re-slurry it is intended that a solid compound is suspended in a solvent or in mixture of solvents. The solid obtained is then usually isolated by filtration or centrifugation.

According to a preferred aspect of the process of the present invention, in step a1) the solvent is an ether.

According to a preferred aspect of the process of the present invention, in step a1) the solvent is tetrahydrofuran, 2-methyl-tetrahydrofuran or tert-butyl-methyl ether.

According to a more preferred aspect of the process of the present invention, in step a1) the solvent is tert-butyl-methyl ether.

According to a preferred aspect of the process of the present invention, in step a1) the solvent is comprised in the range from 2 volumes to 30 volumes with respect to compound of formula (III).

According to a more preferred aspect of the process of the present invention, in step a1) the solvent is comprised in the range from 4 volumes to 20 volumes with respect to compound of formula (III).

As intended herein, the term "volume" (V) means volume of solvent per unit of product, thus, for example, 1 volume is 1 Liter per 1 Kilo, or 1 mL per 1 gram, or 1 microliter per 1 milligram. Thus, 10 volumes means for example 10 liters per 1 Kilogram of substance.

According to a preferred aspect of the process of the present invention, before step a) is comprised the additional step a0) comprising reacting compound of formula (IV): 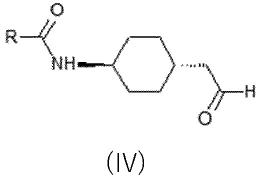 wherein R is O-R₁ or N(CH₃)₂ and wherein R₁ is C₁-C₆ linear or branched alkyl, phenyl or benzyl, with compound of formula (V): 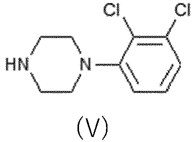 or salts thereof, to give the compound of formula (II): 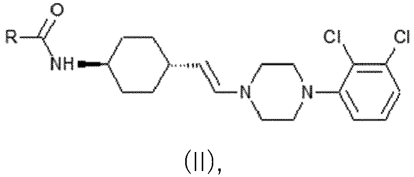 wherein R has the same meaning as above.

According to a more preferred aspect of the process of the present invention step a0) and step a) are carried out as a one-pot reaction.

The present invention is also addressed to the use of formic acid for the preparation of compound of formula (III): 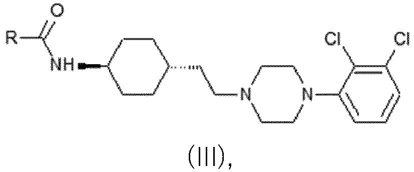 wherein R is O-R₂ or N(CH₃)₂ and wherein R₁ is C₁-C₆ linear or branched alkyl, phenyl or benzyl.

According to another aspect, is preferred the use when formic acid is used for reacting compound of formula (II) to provide compound of formula (III), which is then converted to Cariprazine of formula (I).

All the features and preferred aspects of the process of the present invention given above can be combined in each possible combination to carry out the claimed process.

### Experimental Section

The starting material compound of formula (V) (1-(2,3-dichlorophenyl) piperazine) can be prepared, for example, according to Example 1 of WO 2005012266 (page 18).

Compound of formula (IV) wherein R is N(CH₃)₂ (N,N-dimethyl-N'-[4-(2-oxoethyl)cyclohexyl]urea) can be prepared, for example, according to Example 1 of CN104496854 (pages 10-11).

Compound of formula (IV) wherein R is O-tertbutyl (tert-butyl [4-(2-oxoethyl)cyclohexyl]carbamate) can be prepared for example as reported in WO 2010031735 (synthesis of Intermediate A pages 125-126). Same procedure can be applied for the preparation of compound of formula (IV) wherein R is O-R₂ and wherein R₁ is C₁-C₆ linear or branched alkyl, phenyl or benzyl, by replacing in Step 3 the reagent di-tertbutyl-dicarbonate with the anhydride corresponding to the desired R₁ group.

Room temperature (RT) means a temperature that is comprised in a range from 20 to 25° C, it is defined as comfortable temperature range indoors.

As intended herein, drying the wet material under *vacuum* means to heat the material to a given temperature for a given time while applying a reduced pressure. This operation is performed to remove residual solvents from the product.

As intended herein, to concentrate a mixture under *vacuum* means to heat the material to a given temperature for a given time while applying a reduced pressure. This operation is performed to remove solvents from the mixture.

Example 1: Preparation of trans-[4-[2-[4-(2,3-Dichlorophenyl)piperazin -1-yl]ethyl]cyclohexyl]carbamic acid (compound of formula (III) wherein R is O-tertbutyl).

In a round bottom flask equipped with a half-moon stirrer were charged: 0.62g of 1-(2,3-dichlorophenyl)piperazine (compound of formula (V)) hydrochloride, 15mL of dichloromethane, 7.5 mL of a saturated sodium chloride aqueous solution, 10 mL of a saturated sodium hydrogencarbonate aqueous solution and 0.5mL of sodium hydroxide 30%wt aqueous solution. The mixture was stirred at RT for 10 minutes, then the phases were allowed to settle and separated. The organic layer was washed with 10 mL of saturated sodium chloride aqueous solution. The mixture was stirred at RT for 10 minutes, then the phases were allowed to settle and separated. The organic layer was concentrated to residue under *vacuum.* The residue was taken up with 10 mL of toluene and 0.5g of tert-butyl [4-(2-oxoethyl)cyclohexyl]carbamate (compound of formula (IV)wherein R is O-tertbutyl) were added. The mixture was heated to reflux while separating water with a Dean Stark apparatus for 50 minutes. After cooling down the mixture to T=75-78° C, a solution of 0.4g of formic acid in 1 mL of 2-methyl-tetrahydrofuran was dosed over 1 hour. The mixture was stirred at T=75-78° C for 50 minutes, then cooled down to RT and diluted with 20 mL of toluene and 10 mL of a saturated sodium hydrogencarbonate aqueous solution. After 2 minutes stirring at RT the phases were allowed to settle and separated. The organic layer was washed with 10 ml of a sodium hydroxide 5%wt acqueous solution, then with 5 mL of water and finally with 5 ml of a saturated sodium chloride aqueous solution. The organic layer was anhydrified by treatment with magnesium sulfate, which was filtered washing the cake with toluene. The resulting solution was concentrated to residue under *vacuum.*

0.8g of trans-[4-[2-[4-(2,3-Dichlorophenyl) piperazin-1-yl]ethyl]cyclo hexylcarbamic acid were obtained (84% molar yield, 87% A/A% by HPLC).

Example 2: Preparation of trans-[4-[2-[4-(2,3-Dichlorophenyl)piperazin -1-yl]ethyl]cyclohexyl]carbamic acid (compound of formula (III) wherein R is O-tertbutyl).

In a round bottom flask equipped with a half-moon stirrer were charged: 8.85g of 1-(2,3-dichlorophenyl)piperazine (compound of formula (V)) hydrochloride, 30mL of dichloromethane, 15 mL of a saturated sodium chloride aqueous solution, 20 mL of a saturated sodium hydrogencarbonate aqueous solution and 1mL of sodium hydroxide 30%wt aqueous solution. The mixture was stirred at RT for 10 minutes, then the phases were allowed to settle and separated. The organic layer was washed with 20 mL of saturated sodium chloride aqueous solution. The mixture was stirred at RT for 10 minutes, then the phases were allowed to settle and separated. The organic layer was concentrated to residue under *vacuum.* The residue was taken up with 15 mL of toluene and transferred in a round bottom flask equipped with a half moon stirrer containing 1.0g of tert-butyl [4-(2-oxoethyl)cyclohexyl]carbamate (compound of formula (IV) wherein R is O-tertbutyl) and 5 mL of toluene. The mixture was heated to reflux while separating water with a Dean Stark apparatus for 2 hours. After cooling down the mixture to T=75° C over 15 minutes, the solution was subdivided into two equal portions. One of the two portions was employed in Example 3. A solution of 0.4g of formic acid in 1 mL of 2-methyl-tetrahydrofuran was added on the remaining second portion of the solution. The resulting mixture was stirred at T=75-78° C for 1 hour, then cooled down to RT and diluted with 10 mL of a saturated sodium hydrogencarbonate aqueous solution.

After 2 minutes stirring at RT the phases were allowed to settle and separated. The organic layer was washed with 50 ml of a hydrochloric acid 1%wt acqueous solution, then with 2x30 mL of saturated sodium hydrogencarbonate aqueous solution and finally with 2x20 ml of a saturated sodium chloride aqueous solution. The organic layer was concentrated to residue under *vacuum.*

0.73g of trans-[4-[2-[4-(2,3-Dichlorophenyl) piperazin-1-yl]ethyl]cyclo hexyl]carbamic acid were obtained (79% molar yield, 75% A/A% by HPLC).

Example 3: Preparation of trans-[4-[2-[4-(2,3-Dichlorophenyl)piperazin -1-yl]ethyl]cyclohexyl]carbamic acid (compound of formula (III) wherein R is O-tertbutyl).

In a round bottom flask equipped with a half-moon stirrer was charged the first portion of the solution obtained in Example 2 (refer to paragraph above) and the material was heated to T=75° C. Maintaining this temperature, a solution of 0.4g of formic acid in 1 mL of 2-methyl-tetrahydrofuran was added, followed by 10 microliters of hydrochloric acid 35%wt aqueous solution. The mixture was stirred at T=75-78° C for 1 hour and 45 minutes, then cooled down to T=20° C over 15 minutes. The mixture was then diluted with 10 mL of a saturated sodium hydrogencarbonate aqueous solution. After 2 minutes stirring at RT the phases were allowed to settle and separated. The organic layer was washed with 10 ml of water and then anhydrified by treatment with magnesium sulfate, which was filtered off, washing the cake with toluene. The resulting solution was concentrated to residue under *vacuum.*

0.92g of trans-[4-[2-[4-(2,3-Dichlorophenyl)piperazin-1-yl]ethyl]cyclo hexyl]carbamic acid were obtained (97% molar yield, 75% A/A% by HPLC).

Example 4: Preparation of N' -[trans-4-[2-[4-(2,3-Dichlorophenyl)-1-piperazinyl]ethyl]cyclohexyl]-N,N-dimethylurea (compound of formula (I), Cariprazine).

Product obtained in Example 1, Example 2 or Example 3 can be converted into Compound of formula (I) (Cariprazine), for example according to the procedure reported in WO 2005012266 (Example 3 and Method A at pages 19-20)

Example 5: Preparation of N' -[trans-4-[2-[4-(2,3-Dichlorophenyl)-1-piperazinyl]ethyl]cyclohexyl]-N,N-dimethylurea (compound of formula (I), Cariprazine).

In a round bottom flask equipped with a half-moon stirrer were charged: 0.26g of 1-(2,3-dichlorophenyl)piperazine (compound of formula (V)) hydrochloride, 9 mL of toluene and 10 mL of a saturated sodium hydrogencarbonate aqueous solution. The mixture was stirred at RT for 10 minutes, then the phases were allowed to settle and separated. The organic layer was washed with 10 mL of saturated sodium chloride aqueous solution. The mixture was stirred at RT for 10 minutes, then the phases were allowed to settle and separated. The organic layer was concentrated to residue under *vacuum.* The residue was taken up with a solution of 0.3g of N,N-dimethyl-N'-[4-(2-oxoethyl)cyclohexyl]urea (compound of formula (IV) wherein R is N(CH₃)₂), 5 mL of toluene and 5 mL of dichloromethane. The mixture was heated to reflux while separating water with a Dean Stark apparatus for 50 minutes. After cooling down the mixture to T=72-74° C, 0.19 mL of formic acid were charged. The mixture was stirred at T=75-78° C for 1.5h minutes, then cooled down to RT and concentrated to residue under *vacuum.*

The residue was taken up with 40 mL of dichloromethane and 35 mL of a saturated sodium hydrogen carbonate aqueous solution. The phases were allowed to settle and separated. The organic layer was washed again with 2x 35 mL of a saturated sodium hydrogen carbonate aqueous solution and then anhydrified by treatment with magnesium sulfate, which was filtered off, washing the cake with toluene. The resulting solution was concentrated to residue under *vacuum.*

0.2g of N' -[trans-4-[2-[4-(2,3-Dichlorophenyl)-1-piperazinyl]ethyl] cyclohexyl]-N,N-dimethylurea were obtained (40% molar yield, 91% A/A% by HPLC).

Example 6: Re-crystallization of trans-[4-[2-[4-(2,3-Dichlorophenyl) piperazin-1-yl]ethyl]cyclohexyl]carbamic acid (compound of formula (III) wherein R is O-tertbutyl) in methyl-tert-butyl ether.

In a round bottom flask equipped with a half-moon stirrer were charged: 1.3g of trans-[4-[2-[4-(2,3-Dichlorophenyl)piperazin-1-yl]ethyl]cyclohexyl]carbamic acid (compound of formula (III) wherein R is O-tertbutyl) and 30 mL of methyl-tert-butyl ether. The mixture was heated to 50° C for 10 minutes (complete dissolution observed) and then cooled down to T=0/5° C over 3 hours. After 30 minutes stirring at this temperature, the slurry was filtered and the wet solid was dried under *vacuum.* 0.79g of trans-[4-[2-[4-(2,3-Dichlorophenyl)piperazin-1-yl]ethyl]cyclohexyl] carbamic acid were obtained (61% molar yield, 99% A/A% by HPLC).

Example 7: Re-slurry of trans-[4-[2-[4-(2,3-Dichlorophenyl) piperazin-1-yl]ethyl]cyclohexyl]carbamic acid (compound of formula (III) wherein R is O-tertbutyl) in methyl-tert-butyl ether.

In a round bottom flask equipped with a half-moon stirrer were charged: 2.2g of trans-[4-[2-[4-(2,3-Dichlorophenyl)piperazin-1-yl]ethyl] cyclo hexyl]carbamic acid (compound of formula (III) wherein R is O-tertbutyl) and 10 mL of methyl-tert-butyl ether. The mixture was stirred at RT for 3 hours, then the slurry was filtered washing the cake with 10 mL of methyl-tert-butyl ether and the wet solid was dried under *vacuum.*

1.45g of trans-[4-[2-[4-(2,3-Dichlorophenyl)piperazin-1-yl]ethyl] cyclo hexyl]carbamic acid were obtained (67% molar yield, 98% A/A% by HPLC).

## Claims

1. Process for the preparation of Cariprazine compound of formula (I): 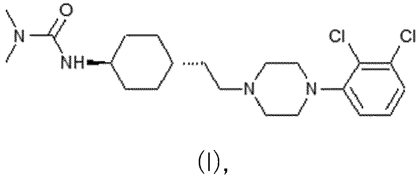 comprising the following steps:
a) reduction reaction of the compound of formula (II): 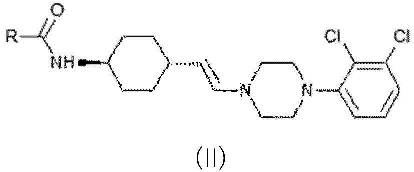 wherein R is O-R₂ or N(CH₃)₂ and wherein R₁ is C₁-C₆ linear or branched alkyl, phenyl or benzyl, with formic acid to give a compound of formula (III): 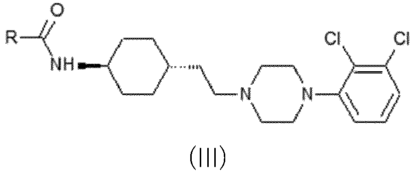 wherein R is O-R₂ or N(CH₃)₂ and wherein R₁ is C₁-C₆ linear or branched alkyl, phenyl or benzyl;
b) when R is not N(CH₃)₂, conversion of compound of formula (III) obtained in step a) to give Cariprazine compound of formula (I):

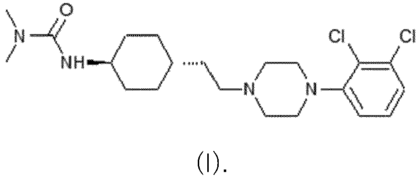

2. Process according to claim 1, wherein R is O-tertbutyl.

3. Process according to any one of the claims 1 or 2, wherein in step a) the reduction reaction is carried out in a solvent and wherein the solvent is toluene, ethanol or 2-Methyl-tetrahydrofuran.

4. Process according to any one of the claims from 1 to 3, wherein in step a) the reaction is carried out at a temperature comprised in the range from 60° C to 80° C.

5. Process according to any one of the claims from 1 to 4, wherein in step a) the reaction is carried out for a time comprised in the range from 30 minutes to 4 hours.

6. Process according to any one of the claims from 1 to 5, wherein in step a) the reaction is carried out with an amount of formic acid comprised in the range from 1 molar equivalent to 10 molar equivalents or from 3 molar equivalents to 5 molar equivalents with respect to compound of formula (II).

7. Process according to any one of the claims from 1 to 6, wherein step a) is performed in the presence of an inorganic acid or in step a) an inorganic acid is also added.

8. Process according to any one of the claims from 1 to 7, wherein in step a) the formic acid is added as a solution in an organic solvent.

9. Process according to any one of the claims from 1 to 8, wherein in step a) the formic acid is added neat into the reaction mixture.

10. Process according to any one of the claims from 1 to 9, wherein between step a) and step b) is comprised an additional step a1) comprising a re-crystallization or re-slurry of compound of formula (III) in an organic solvent.

11. Process according to claim 10 wherein the organic solvent is MTBE.

12. Process according to any one of the claims 10 or 11, wherein the solvent is comprised in the range from 2 volumes to 30 volumes or from 4 volumes to 20 volumes with respect to compound of formula (III).

13. Process according to any one of the claims from 1 to 12, further comprising before step a) the additional step a0) comprising reacting compound of formula (IV): 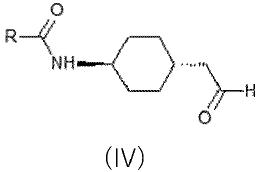 wherein R is O-R₁ or N(CH₃)₂ and wherein R₁ is C₁-C₆ linear or branched alkyl, phenyl or benzyl, with compound of formula (V): 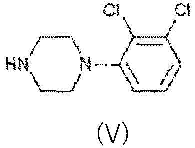 or salts thereof, to give the compound of formula (II):

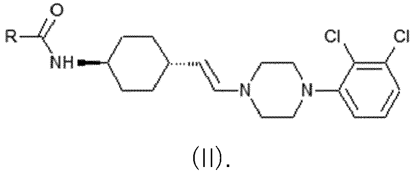

14. Process according to claim 13, wherein step a0) and step a) are carried out as an one-pot reaction.

15. Use of formic acid for the preparation of the compound of formula (III): 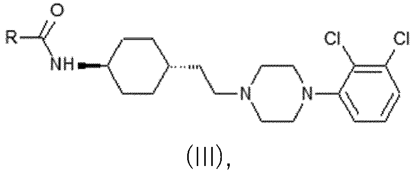 wherein R is O-R₁ or N(CH₃)₂ and wherein R₁ is C₁-C₆ linear or branched alkyl, phenyl or benzyl.

## Amended claims

### Amended claims in accordance with Rule 137(2) EPC.

1. Process for the preparation of Cariprazine compound of formula (I): 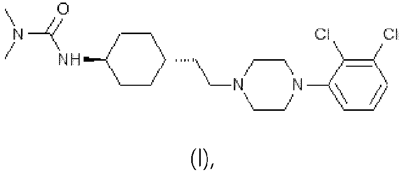 comprising the following steps:
a) reduction reaction of the compound of formula (II): 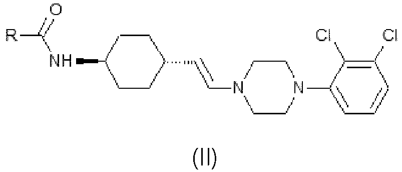 wherein R is O-R₁ or N(CH₃)₂ and wherein R₁ is C₁-C₆ linear or branched alkyl, phenyl or benzyl, with formic acid to give a compound of formula (III): 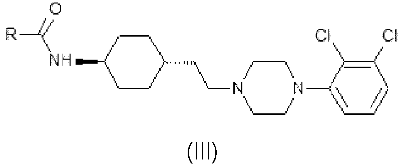 wherein R is O-R₁ or N(CH₃)₂ and wherein R₁ is C₁-C₆ linear or branched alkyl, phenyl or benzyl;
b) when R is not N(CH₃)₂, conversion of compound of formula (III) obtained in step a) to give Cariprazine compound of formula (I):

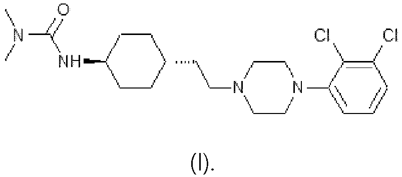

2. Process according to claim 1, wherein R is O-tertbutyl.

3. Process according to any one of the claims 1 or 2, wherein in step a) the reduction reaction is carried out in a solvent and wherein the solvent is toluene, ethanol or 2-Methyl-tetrahydrofuran.

4. Process according to any one of the claims from 1 to 3, wherein in step a) the reaction is carried out at a temperature comprised in the range from 60°C to 80°C.

5. Process according to any one of the claims from 1 to 4, wherein in step a) the reaction is carried out for a time comprised in the range from 30 minutes to 4 hours.

6. Process according to any one of the claims from 1 to 5, wherein in step a) the reaction is carried out with an amount of formic acid comprised in the range from 1 molar equivalent to 10 molar equivalents or from 3 molar equivalents to 5 molar equivalents with respect to compound of formula (II).

7. Process according to any one of the claims from 1 to 6, wherein step a) is performed in the presence of an inorganic acid or in step a) an inorganic acid is also added.

8. Process according to any one of the claims from 1 to 7, wherein in step a) the formic acid is added as a solution in an organic solvent.

9. Process according to any one of the claims from 1 to 8, wherein in step a) the formic acid is added neat into the reaction mixture.

10. Process according to any one of the claims from 1 to 9, wherein between step a) and step b) is comprised an additional step a1) comprising a re-crystallization or re-slurry of compound of formula (III) in an organic solvent.

11. Process according to claim 10 wherein the organic solvent is MTBE.

12. Process according to any one of the claims 10 or 11, wherein the solvent is comprised in the range from 2 volumes to 30 volumes or from 4 volumes to 20 volumes with respect to compound of formula (III).

13. Process according to any one of the claims from 1 to 12, further comprising before step a) the additional step a0) comprising reacting compound of formula (IV): 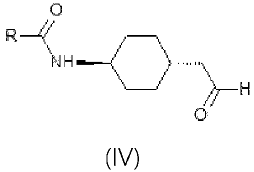
wherein R is O-R₁ or N(CH₃)₂ and wherein R₁ is C₁-C₆ linear or branched alkyl, phenyl or benzyl, with compound of formula (V): 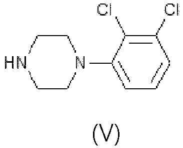
or salts thereof, to give the compound of formula (II):

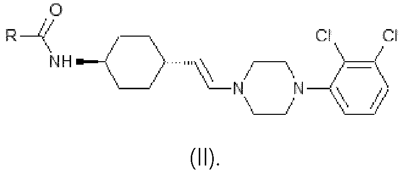

14. Process according to claim 13, wherein step a0) and step a) are carried out as an one-pot reaction.

15. Use of formic acid for the preparation of the compound of formula (III): 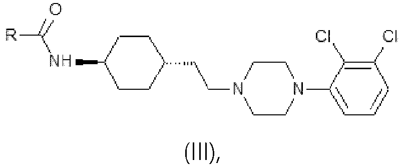
wherein R is O-R₁ or N(CH₃)₂ and wherein R₁ is C₁-C₆ linear or branched alkyl, phenyl or benzyl,
wherein formic acid is used for reacting compound of formula (II) to provide compound of formula (III),which is then converted to Cariprazine of formula (I).
